(19) Europäisches Patentamt
European Patent Office
Office européen des brevets

(11) Numéro de publication: **0 060 176**
**B1**

(12) # FASCICULE DE BREVET EUROPEEN

(45) Date de publication du fascicule du brevet: **02.01.85**

(51) Int. Cl.³: **C 07 D 211/70**, A 61 K 31/445

(21) Numéro de dépôt: **82400320.6**

(22) Date de dépôt: **24.02.82**

(54) **Trifluorométhylphényltétrahydropyridines substituées à activité anorexigène, un procédé de préparation et compositions pharmaceutiques.**

(30) Priorité: **11.03.81 FR 8104890**

(43) Date de publication de la demande: **15.09.82 Bulletin 82/37**

(45) Mention de la délivrance du brevet: **02.01.85 Bulletin 85/1**

(84) Etats contractants désignés: **AT BE CH DE FR GB IT LI LU NL SE**

(56) Documents cités:
**DE - A - 2 101 997**
**FR - E - 86 403**
**GB - A - 881 894**
**GB - A - 948 071**
**US - A - 3 125 488**
**US - A - 3 284 457**

(73) Titulaire: **SANOFI, société anonyme, 40, Avenue George V, F-75008 Paris (FR)**

(72) Inventeur: **Nisato, Dino, Viale Golgi 80, Pavia (IT)**
Inventeur: **Crisafulli, Emilio, Viale San Gimigrano 12, I-20146 Milan (IT)**
Inventeur: **Bianchetti, Alberto, Via F. Corridoni 11, I-20122 Milan (IT)**
Inventeur: **Carminati, Paolo, Via Pacini 24, Milan (IT)**

(74) Mandataire: **Gillard, Marie-Louise et al, Cabinet Beau de Loménie 55, Rue d'Amsterdam, F-75008 Paris (FR)**

**EP 0 060 176 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne de nouveaux composés à activité anorexigène.

Plus particulièrement, l'invention se réfère à de nouvelles 4-(3-trifluorométhylphényl)-1,2,3,6-tétrahydropyridines de formule

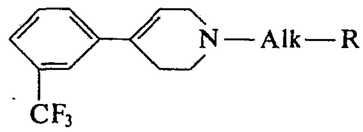

(I)

dans laquelle R représente un groupe cyano, acétyle ou cycloalkyle contenant 3 à 7 atomes de carbone et Alk représente un groupe alkylène à chaîne droite ou ramifiée contenant de 1 à 4 atomes de carbone, ainsi que leurs sels pharmaceutiquement acceptables, dotés d'une activité anorexigène remarquable.

Le brevet GB-A-881 894 décrit une série de 1-aroylalkyl-4-aryl-1,2,3,6-tétrahydropyridines de formule

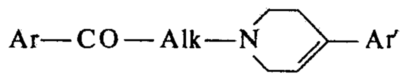

(II)

et de ses sels non toxiques pharmaceutiquement utiles, où Ar et Ar' sont chacun un radical halophényle, alkoxyphényle contenant moins de 11 atomes de carbone, ou un radical diméthoxyphényle, hydroxyphényle, thiényle, trifluorométhylphényle ou aromatique monocyclique contenant moins de 11 atomes de carbone et Alk est un radical alkylène contenant de 3 à 6 atomes de carbone.

Un des procédés de préparation des composés II ci-dessus prévoit la réaction d'un $\omega$-(4-aryl-1,2,3,6-tétrahydropyridine)alcanonitrile approprié avec un halogénure d'arylmagnésium, la décomposition du complexe résultant et l'isolation du produit.

Le brevet ci-dessus suggère donc la possibilité d'utiliser certains $\omega$-(4-aryl-1,2,3,6-tétrahydropyridine)alcanonitriles comme produits intermédiaires dans la préparation de composés à action anticonvulsivante, déprimant du système nerveux central et tranquilisante. Il ne décrit cepencant aucune 3-trifluorométhylphényl-1,2,3,6-tétrahydropyridine substituée par un groupe cyanoalkyle.

Il est connu que le chef de file des composés à action anorexigène est l'amphétamine qui exerce son activité par un mécanisme d'action biochimique central au niveau des systèmes dopaminergique et noradrénergique.

L'amphétamine et ses dérivés présentent des inconvénients importants car leur effet stimulant le système nerveux central ainsi que la possibilité d'accotumance et de pharmacodépendance peuvent constituer un danger potentiel pour le patient.

Des études ont été donc consacrées à la recherche de dérivés de l'amphétamine présentant une dissociation entre l'effet stimulant et l'action anorexigène. L'introduction d'un groupe trifluorométhyle dans la position méta du groupe phényle de l'éthylamphétamine a donné lieu à l'obtention d'un produit, ci-après désigné par sa Denomination Commune Internationale »fenfluramine«, doté d'une excellente activité anorexigène, qui, au lieu d'être stimulant, possède une certaine action sédative.

L'avantage de la fenfluramine et de ses dérivés par rapport à l'amphétamine et à ses dérivés est dû au différent mécanisme d'action. En fait, l'anorexie provoquée par l'amphétamine semble être médiée par le »release« de noradrénaline cérébrale, alors que l'action anorexigène de la fenfluramine dépend du »release« de la sérotonine endogène des neurones centraux (Ann. C. Sullivan et al. Appetite Regulation and Its Modulation by Drugs. Nutrition and drug interrelation, 1978, Academic Press, 21—82) et de l'inhibition de l'uptake de la sérotonine.

Il est cependant connu que la fenfluramine, à des doses très proches à la dose anorexigène, provoque une réduction significative des taux cérébraux de sérotonine (Arch. Intern. Pharmacodyn. Ther. 1967, 170, 276) et qu'une déplétion durable de sérotonine peut être considérée comme un signe de neurotoxicité potentielle (C.D. Morgan et al. Life Sci. Part I, 11, 83; 1972).

On a maintenant trouvé que les 4-(3-trifluorométhylphényl)-1,2,3,6-tétrahydropyridines de formule I ci-dessus, et leurs sels, montrent une activité anorexigène remarquable associée à une très basse toxicité. Sur le plan biochimique, les composés de formule I ci-dessus, ainsi que leurs sels, agissent en tant qu'agonistes de la sérotonine cérébrale sans provoquer aucune déplétion de sérotonine cérébrale ni stimulation du système nerveux central. Plus particulièrement, les composés de formule I ci-dessus montrent une grande affinité pour les récepteurs post-synaptiques de la sérotonine et, par cette stimulation directe du système sérotoninergique, ils entrainent une activité anorexigène sans les effets secondaires dus au release de sérotonine.

Ainsi, la présente invention a pour objet les nouvelles 4-(3-trifluorométhylphényl)-1,2,3,6-tétrahydropyridines de formule I ci-dessus, ainsi que leurs sels pharmaceutiquement acceptables.

Les sels pharmaceutiquement acceptables comprennent les sels non toxiques dérivés d'acides minéraux ou organiques tels que le chlorhydrate, le bromhydrate, le succinate, le tartrate, le citrate, le fumarate, le maléate, le 4,4'-méthylènebis-(3-hydroxy-2-naphtoate), ci-après désigné »pamoate«, le

2-naphtalènesulfonate, ci-après désigné »napsylate«, le méthanesulfonate, ci-après désigné »mésylate« et le p-toluènesulfonate, le ci-après désigné »tosylate«.

Selon un autre de ses aspects, la présente invention se réfère à un procédé pour la préparation des 4-(3-trifluorométhylphényl)-1,2,3,6-tétrahydropyridines de formule I ci-dessus et de leurs sels.

Ledit procédé est caractérisé en ce qu'on fait réagir, dans un solvant organique et à une température de 20 à 200°C, la 4-(3-trifluorométhylphényl)-1,2,3,6-tétrahydropyridine de formule

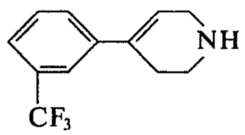

(III)

avec un composé de formule X – R, où R a la signification donnée ci-dessus et X représente un groupe chloro-, bromo- ou iodoalkyle ayant 1 à 4 atomes de carbone ou bien, seulement dans le cas où R est cyano ou acétyle, un groupe vinyle non substitué ou substitué par 1 ou 2 groupes méthyle ou par un groupe éthyle.

La 4-(3-trifluorométhylphényl)-1,2,3,6-tétrahydropyridine utilisée comme produit de départ est décrite dans les brevets français 1 421 208 et 1 569 013.

Comme solvant organique préférentiel, on utilise un alcool aliphatique ayant 1 à 6 atomes de carbone, tel que méthanol, éthanol, n-butanol, n-pentanol, mais d'autres solvants tels que hexane, diméthylformamide, diméthylsulfoxyde, sulfolane, acétonitrile ou pyridine peuvent être utilisés.

La réaction est avantageusement conduite en présence d'un agent de condensation basique, tel que la triéthylamine, surtout dans le cas où le réactif R – X est un dérivé halogéné.

La température de réaction peut varier entre la température ambiante (environ 20°C) et 200°C et sa durée varie de consequence. En générale, après 4 à 5 heures de chauffage à 100–150°C, la réaction est complète et le produit final ainsi obtenu peut être isolé selon les techniques conventionnelles et éventuellement transformé dans ses sels par traitement avec une solution de l'acide choisi dans un solvant organique.

Selon un autre de ses aspects, la présente invention concerne un procédé de préparation des composés de formule I ci-dessus dans laquelle Alk est à chaîne droite et R est autre que cyano et acétyle, à savoir de composés de formule

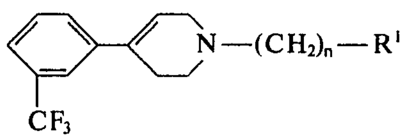

(IV)

où $R^1$ représente un groupe cycloalkyle contenant 3 à 7 atomes de carbone et n est 1, 2, 3 ou 4, caractérisé en ce qu'on réduit un composé de formule

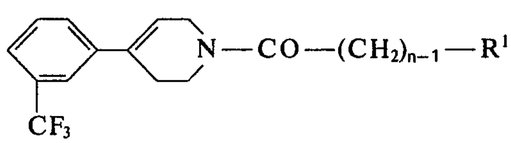

(V)

dans laquelle $R^1$ et n sont tels que définis ci-dessus, par un hydrure d'aluminium ou par un hydrure complexe de lithium et d'aluminium dans un solvant organique inerte à une température de 0°C à la température d'ébullition du solvant employé et l'on transforme éventuellement le produit ainsi obtenu dans ses sels pharmaceutiquement acceptables.

La réaction de réduction est conduite selon les modes opérationnels connus en soi en utilisant comme agent de réduction l'hydrure d'aluminium ou un hydrure complexe de lithium et d'aluminium, tel que $LiAlH_4$ ou $LiAlH(OCH_3)_3$. On opère en général dans un solvant inerte comme un éther, tel que l'éther diéthylique, le tétrahydrofuranne, le dioxanne ou le 1,2-diméthoxyéthane.

Selon un mode opérationnel préférentiel, on opère avec une quantité équimoléculaire d'hydrure de lithium et d'aluminium $LiAlH_4$ par rapport au composé V de départ, à une température de 20–30°C dans de l'éther diéthylique et sous atmosphère inerte. Après environ une heure, la réduction est complète et le composé de formule IV est isolé selon les techniques conventionnelles sous forme de base libre ou d'un de ses sels.

La base libre ainsi obtenue peut être transformée dans un de ses sels par simple salification dans un solvant organique tel qu'un alcool, éthanol et isopropanol de préférence, un éther comme le 1,2-diméthoxyéthane, l'acétate d'éthyle ou un hydrocarbure comme l'hexane.

Les composés de formule V ci-dessus sont préparés en faisant réagir la 4-(3-trifluorométhylphényl)-1,2,3,6-tétrahydropyridine de formule III avec un dérivé fonctionnel d'un acide carboxylique de formule

$$R^1 - (CH_2)_{n-1} - COOH \qquad (VI)$$

où $R^1$ et n sont tels que définis ci-dessus, dans un solvant organique à une température comprise entre − 10°C et la température d'ébullition du solvant employé.

Comme dérivé approprié, on peut utiliser l'anhydride, une anhydride mixte, un ester activé ou un halogènure d'acide, le chlorure de préférence. Parmi les esters activés, l'ester de nitrophényle est particulièrement préféré, mais l'ester de méthoxyphényle, de trityle ou de benzhydrile sont également convenables.

La température de réaction peut varier entre 0°C et le point d'ébullition du solvant employé, mais en général on opère à la température ambiante ou à 30–50°C. Il peut être préférable de conduire la réaction au froid lorsqu'elle est exothermique, comme dans le cas où on utilise le chlorure en tant que dérivé fonctionnel de l'acide benzoïque de formule VI.

Comme solvant de réaction on utilise de préférence un alcool, tel que le méthanol ou l'éthanol, ou un solvant halogéné, tel que le chlorure de méthylène, le dichloroéthane, le chloroforme et similaires, mais d'autres solvants organiques compatibles avec les réactifs employés, par exemple le dioxanne, le tétrahydrofuranne ou un hydrocarbure tel que l'hexane peuvent également être employés.

La réaction peut être conduite en présence d'un accepteur de protons, par exemple d'un carbonate alcalin ou d'une amine tertiaire, dans le cas où de l'acide chlorhydrique, ou un autre acide, se libère pendant la reaction, mais cet accepteur de protons n'est pas indispensable pour l'obtention du produit final.

Le produit que l'on obtient à la fin de la réaction est en générale une huile qui peut être isolée et caractérisée selon les techniques conventionnelles, mais qui peut être utilisée à l'état brut pour la réduction avec l'hydrure.

Les 4-(3-trifluorométhylphényl)-1,2,3,6-tétrahydropyridines de la présente invention et leurs sels montrent une activité anorexigène remarquable et sélective sans donner aucun effet du type amphétaminique. La sélectivité de leur action est démontrée par la manque d'activités pharmacologiques secondaires, telles que l'activité sédative ou excitante et l'action inhibitrice de l'activité locomotrice.

L'activité anorexigène a été évaluée par la méthode de la prise de nourriture chez le rat. On a utilisé des rats femelles de 200 g entrainés pendant 10 jours à manger durant une période de 4 heures et sélectionnés au huitième jour. Au bout du dixième jour, les animaux randomisés ont été divisés en un »groupe contrôle«, traité par le seul véhicule, et en plusieurs »groupes traités«. Le traitement a été effectué par voie intrapéritonéale ou orale 30 minutes ou 1 heure avant la présentation de la nourriture et, ensuite, on a mesuré la quantité de nourriture consommée au cours de la première heure.

Dans le tableau I sont reportés, pour sept composés réprésentatifs de l'invention:

- la toxicité aiguë, exprimée comme $DL_{50}$ chez le rat par voie orale ou par voie intrapéritonéale (donnée A);
- l'activité anorexigène, exprimée comme dose orale ou intrapéritonéale inhibant de 50% la prise de nourriture ($DI_{50}$, donnée B);
- le rapport entre la toxicité aiguë et l'activité anorexigène, qui exprime l'index thérapeutique lié à la toxicité aiguë (donnée A/B).

Comme produits représentatifs de la présente invention on a utilisé les composés suivants:

- le chlorhydrate de 4-(3-trifluorométhylphényl)-1-(2-cyanoéthyl)-1,2,3,6-tétrahydropyridine (composé de l'Exemple 2),
- le chlorhydrate de 4-(3-trifluorométhylphényl)-1-(3-cyanoéthyl)-1,2,3,6-tétrahydropyridine (composé de l'Exemple 3),
- le chlorhydrate de 4-(3-trifluorométhylphényl)-1-(4-cyanoéthyl)-1,2,3,6-tétrahydropyridine (composé de l'Exemple 4),
- le chlorhydrate de 4-(3-trifluorométhylphényl)-1-acétonyl-1,2,3,6-tétrahydropyridine (composé de l'Exemple 5),
- le chlorhydrate de 4-(3-trifluorométhylphényl)-1-(3-oxobutyl)-1,2,3,6-tétrahydropyridine (composé de l'Exemple 6),
- le chlorhydrate de 4-(3-trifluorométhylphényl)-1-cyclopropylméthyl)-1,2,3,6-tétrahydropyridine (composé de l'Exemple 7),
- le chlorhydrate de 4-(3-trifluorométhylphényl)-1-(2-cyclohexyléthyl)-1,2,3,6-tétrahydropyridine (composé de l'Exemple 10).

Tableau I

| Composé | Voie d'administration | A<br>$DL_{50}$<br>mg/kg | B<br>$DI_{50}$<br>mg/kg | A/B |
|---|---|---|---|---|
| Exemple 2 | i.p. | 205,8 | 12,0 | 17,1 |
| | os | 254,2 | 9,5 | 26,7 |
| Exemple 3 | i.p. | 258,5 | 11,1 | 23,3 |
| | os | 538,5 | 11,7 | 46 |
| Exemple 4 | os | 335,6 | 10,5 | 32,0 |
| Exemple 5 | i.p. | 165,0 | 10,8 | 15,3 |
| Exemple 6 | i.p. | 134,2 | 6,5 | 20,6 |
| | os | 298,6 | 11,8 | 25,3 |
| Exemple 7 | i.p. | 135,4 | 5,5 | 24,6 |
| Exemple 10 | os | 488,4 | 2,6 | 187,8 |
| fenfluramine | i.p. | 85,6 | 5,4 | 15,8 |
| | os | 100,4 | 4,0 | 25,1 |

De ce tableau il ressort que les produits représentatifs de la présente invention montrent une bonne activité anorexigène avec une faible toxicité. Leur efficacité est comparable, sur le plan de l'index thérapeutique, à celle du composé de référence.

Sur le plan biochimique, les 4-(3-trifluorométhylphényl)-1,2,3,6-tétrahydropyridines de la présente invention et leurs sels diffèrent de la fenfluramine et de ses dérivés dans leur mécanisme d'action. En fait, les composés de la présente invention sont des agonistes post-synaptiques de la sérotonine avec des effets très faibles sur les mécanismes pré-synaptiques, tels que l'»uptake« et le »release« de la sérotonine, sur lesquels, au contraire, la fenfluramine agit. Le mécanisme d'action des composés de la présente invention comporte une activité anorexigène remarquable et des effets secondaires réduits.

En particulier, les composés de la présente invention, in vivo, ne provoquent pas de déplétion de sérotonine au niveau central. Il y a donc une moindre possibilité que l'usage prolongé des composés de la présente invention provoque des effets secondaires au niveau central.

Le tableau II ci-dessous résume les taux cérébraux de sérotonine, en pourcentage par rapport aux contrôles, après administration intrapéritonéale ou orale de quatre composés représentatifs de la présente invention. La détermination des taux cérébraux, selon Curzon et Green (Brit. J. Pharmacol. 39, 653, 1970), a été effectuée une heure et/ou deux heures après l'administration. La fenfluramine a été utilisée comme produit de référence.

Tableau II

| Composé | Voie d'administration | dose mg/kg | taux cérébraux de sérotonine % par rapport aux contrôles 1 h. | 2 h. |
|---|---|---|---|---|
| Exemple 2 | i.p. | 7,5 | – | 128 |
| | | 15,0 | 131,0 | 146 |
| | | 30,0 | – | 146 |
| Exemple 6 | i.p. | 3,25 | – | 109 |
| | | 7,5 | – | 108 |
| | | 15,0 | – | 116 |
| Exemple 7 | i.p. | 3,25 | – | 111 |
| | | 7,5 | – | 123 |
| | | 15,0 | – | 119 |
| Exemple 10 | os | 1,25 | – | 99 |
| | | 2,5 | – | 95 |
| | | 5 | – | 101 |
| fenfluramine | i.p. | 5 | 82,9*) | – |
| | | 7,5 | 81,6*) | 53,1**) |
| | | 10 | 79,0**) | – |

*) significatif $P < 0,05$.
**) significatif $P < 0,01$.

De ce tableau il ressort que les produits de la présente invention, à une dose supérieure à la $DE_{50}$ anorexigène, ne diminuent pas les taux cérébraux de sérotonine, tandis que la fenfluramine provoque une réduction significative de sérotonine cérébrale.

L'affinité des composés de la présente invention pour les récepteurs post-synaptiques de la sérotonine a été déterminée selon la méthode de Peroutka et Snyder (Molec. Pharmacol. 1979, 16, 687–699) qui consiste à incuber des membranes de cortex de rat avec une concentration fixée de 3H-sérotonine en présence de différentes concentrations de produit. Le tableau III montre la concentration molare de cinq produits représentatifs de la présente invention qui donne une inhibition de 50% du binding spécifique au récepteur sérotoninergique ($IC_{50}$) à savoir la mesure de la capacité du produit d'interagir avec la 3H-sérotonine dans le binding à son récepteur. La fenfluramine a été utilisée comme composé de référence.

Tableau III

| Composé | binding 3H-sérotonine (2 $\mu$M) $IC_{50}$ [M] |
|---|---|
| Exemple 7 | $4,3 \cdot 10^{-7}$ |
| Exemple 3 | $3,1 \cdot 10^{-7}$ |
| Exemple 6 | $1,1 \cdot 10^{-7}$ |
| Exemple 7 | $4,1 \cdot 10^{-7}$ |
| Exemple 10 | $1,8 \cdot 10^{-7}$ |
| fenfluramine | $>10^{-5}$ |

De ce tableau il ressort que les composés de la présente invention ont une très bonne affinité pour le récepteur sérotoninergique post-synaptique alors que l'affinité du composé de référence pour le même récepteur est beaucoup plus faible.

Ainsi, selon un autre de ses aspects, la présente invention se réfère à des compositions pharmaceutiques renfermant, en tant qu'ingrédients actifs, des 4-(3-trifluorométhylphényl)-1,2,3,6-tétrahydropyridines de formule I ci-dessus, ainsi que leurs sels d'addition pharmaceutiquement acceptables.

Dans les compositions pharmaceutiques de la présente invention pour l'administration orale, sublinguale, sous-cutanée, intramusculaire, intraveineuse, transdermique ou rectale, les ingrédients actifs de formule I ci-dessus peuvent être administrés sous formes unitaires d'administration, en mélange avec des supports pharmaceutiques classiques, aux animaux et aux êtres humains pour le traitement de l'obésité. Parmi les formes unitaires d'administration appropriées, il y a les formes par voie orale telles que les comprimés, les gélules, les poudres, les granules et les solutions ou suspensions orales et les formes d'administration sublinguale et buccale, de même que les formes d'administration parentérale utiles pour une administration sous-cutanée, intramusculaire ou intraveineuse.

Afin d'obtenir l'effet anorexigène désiré, la dose de principe actif peut varier entre 0,1 et 100 mg par kg de poids du corps et par jour.

Chaque dose unitaire peut contenir de 1 à 500 mg d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de sucrose ou d'autres matières appropriées ou encore on peut les traiter d'une autre manière de telle sorte qu'ils aient une activité prolongée ou retardée et qu'ils libèrent continuellement une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant acalorique, du méthylparaben et du propylparaben comme antiseptiques, ainsi qu'un agent donnant du goût et un colorant approprié.

Les poudres ou les granulats dispersibles dans l'eau peuvent contenir l'ingrédient actif en mélange avec des agents de dispersion ou des agents mouillants, ou des agents de mise en suspension, tels que la polyvinylpyrrolidone et similaires, de même qu'avec des édulcorants ou des correcteurs du goût.

Pour une application rectale, on recourt à des suppositoires qui sont préparés avec des liants fondant à la température rectale, par exemple du beurre de cacao ou des polyéthylèneglycols.

Pour une application parentérale, on utilise des suspensions aqueuses, des solutions salines isotoniques ou des solutions particulières, stériles et injectables, qui contiennent des agents de dispersion et/ou mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à coté des 4-(3-trifluorométhylphényl)-1,2,3,6-tétrahydropyridines de la présente invention ou d'un de leurs sels pharmaceutiquement acceptables, d'autres principes actifs tels que, par exemple, des tranquilisants, des antidépresseurs, des hypolipémiants, des antidiabétiques ou d'autres médicaments qui peuvent être utilisés dans le traitement de l'obésité.

Les exemples suivants illustrent l'invention sans toutefois la limiter.

## Exemple 1

On chauffe au reflux pendant 5 heures un mélange de 0,025 mole de 4-(3-trifluorométhylphényl)-1,2,3,6-tétrahydropyridine, de 3,5 ml de triéthylamine et 0,025 mole de chloroacétonitrile dans 40 ml d'étanol. On refroidit le mélange réactionnel, on concentre et on reprend le résidu avec de l'éther diéthylique. On filtre la solution éthérée ainsi obtenue, on la lave à l'eau et on la sèche sur du sulfate de sodium anhydre. A la solution ainsi obtenue, on ajoute une solution saturée d'acide chlorhydrique gazeux et isopropanol. On obtient ainsi le chlorhydrate de 4-(3-trifluorométhylphényl)-1-cyanométhyl-1,2,3,6-tétrahydropyridine qui, après cristallisation dans de l'isopropanol fond à 169—172°C. Rendement 75% de la théorie.

## Exemples 2 à 13

En opérant comme décrit à l'Exemple 1, en faisant réagir la 4-(3-trifluorométhylphényl)-1,2,3,6-tétrahydropyridine respectivement avec 3-chloropropionitrile, 4-chlorobutyronitrile, 5-chlorovaléronitrile, chloroacétone, 1-chloro-3-butanone, le chlorure de cyclopropylméthyle, le chlorure de cyclo-

hexylméthyle, le 2-cyclopentyl-1-chloroéthane, le 2-cyclohexyl-1-chloroéthane, le 2-cycloheptyl-1-chloroéthane, le 3-cyclohexyl-1-chloropropane et le 2-chloropropionitrile, on obtient les composés du tableau IV ci-dessous.

Tableau IV

| Exemple | Composé |
|---|---|
| 2 | Chlorhydrate de 4-(3-trifluorométhylphényl)-1-(2-cyanoéthyl)-1,2,3,6-tétrahydropyridine qui, après cristallisation dans de l'éthanol 95°, fond à 226–229°C; rendement: 50% de la théorie. |
| 3 | Chlorhydrate de 4-(3-trifluorométhylphényl)-1-(3-cyanopropyl)-1,2,3,6-tétrahydropyridine qui, après cristallisation dans de l'alcool isopropylique, fond à 168–170°C; rendement: 46,7% de la théorie. |
| 4 | Chlorhydrate de 4-(3-trifluorométhylphényl)-1-(4-cyanobutyl)-1,2,3,6-tétrahydropyridine qui, après cristallisation dans de l'isopropanol, fond à 146–148°C; rendement: 45% de la théorie. |
| 5 | Chlorhydrate de 4-(3-trifluorométhylphényl)-1-acétonyl-1,2,3,6-tétrahydropyridine qui, après cristallisation dans de l'alcool isopropylique, fond à 188–190°C; rendement: 56% de la théorie. |
| 6 | Chlorhydrate de 4-(3-trifluorométhylphényl)-1-(3-oxobutyl)-1,2,3,6-tétrahydropyridine qui, après cristallisation dans de isopropanol, fond à 173–176°C; rendement: 45% de la théorie. |
| 7 | Chlorhydrate de 4-(3-trifluorométhylphényl)-1-cyclopropylméthyl-1,2,3,6-tétrahydropyridine qui, après cristallisation dans de l'acétone, fond à 178–180°C; rendement: 35% de la théorie. |
| 8 | Chlorhydrate de 4-(3-trifluorométhylphényl)-1-cyclohexylméthyl-1,2,3,6-tétrahydropyridine qui, après cristallisation dans un mélange isopropanol-acétate d'éthyle 1:4, fond à 217–220°C; rendement: 45% de la théorie. |
| 9 | Chlorhydrate de 4-(3-trifluorométhylphényl)-1-(2-cyclopentyléthyl)-1,2,3,6-tétrahydropyridine qui, après cristallisation dans de l'isopropanol, fond à 240–245°C; rendement: 39% de la théorie. |
| 10 | Chlorhydrate de 4-(3-trifluorométhylphényl)-1-(2-cyclohexyléthyl)-1,2,3,6-tétrahydropyridine qui, après cristallisation dans de l'isopropanol, fond à 252–257°C; rendement: 48% de la théorie. |
| 11 | Chlorhydrate de 4-(3-trifluorométhylphényl)-1-(2-cycloheptyléthyl)-1,2,3,6-tétrahydropyridine qui, après cristallisation dans de l'isopropanol, fond à 262–265°C; rendement: 40% de la théorie. |
| 12 | Chlorhydrate de 4-(3-trifluorométhylphényl)-1-(3-cyclohexylpropyl)-1,2,3,6-tétrahydropyridine qui, après cristallisation dans de l'acétone, fond à 219–222°C; rendement: 25% de la théorie. |
| 13 | Chlorhydrate de 4-(3-trifluorométhylphényl)-1-(2-cyano-1-méthyl)éthyl-1,2,3,6-tétrahydropyridine. |

## Exemple 14

On chauffe au reflux pendant 4 heures un mélange de 0,03 mole de 4-(3-trifluorométhylphényl)-1,2,3,6-tétrahydropyridine, de 3,8 ml de triéthylamine et 0,03 mole de 3-chloropropionitrile dans 40 ml d'éthanol. On refroidit le mélange réactionnel, on concertre, on reprend le résidu avec de l'éther

diéthylique et on filtre. En traitant la solution éthérée ainsi obtenue, contenant la 4-(3-trifluorométhylphényl)-1-(2-cyanoéthyl)-1,2,3,6-tétrahydropyridine sous forme de base, avec de l'acide p-toluènesulfonique, de l'acide maléique et, respectivement, de l'acide 2-naphtalènesulfonique, on obtient:
le tosylate de 4-(3-trifluorométhylphényl)-1-(2-cyanoéthyl)-1,2,3,6-tétrahydropyridine qui, après cristallisation dans de l'acétone, fond à 154—158°C;

le maléate de 4-(3-trifluorométhylphényl)-1-(2-cyanoéthyl)-1,2,3,6-tétrahydropyridine qui, après cristallisation dans de l'acétone, fond à 85—95°C;
et, respectivement,
le napsylate de 4-(3-trifluorométhylphényl)-1-(2-cyanoéthyl)-1,2,3,6-tétrahydropyridine qui, après cristallisation dans de l'acétone, fond à 178—180°C;

D'une façon analogue on obtient:
le maléate de 4-(3-trifluorométhylphényl)-1-cyclopropylméthyl-1,2,3,6-tétrahydropyridine qui, après trituration dans de l'éther diéthylique, fond à 78—80°C;
le napsylate de 4-(3-trifluorométhylphényl)-1-cyclopropylméthyl-1,2,3,6-tétrahydropyridine qui, après cristallisation dans un mélange eau-acétone, fond à 139—142°C;
et, respectivement,
le tosylate de 4-(3-trifluorométhylphényl)-1-cyclopropylméthyl-1,2,3,6-tétrahydropyridine qui, après cristallisation dans de l'acétone, fond à 86—88°C;

## Exemple 15

On ajoute 0,0263 mole d'acrylonitrile à une solution de 0,025 mole de 4-(3-trifluorométhylphényl)-1,2,3,6-tétrahydropyridine dans 19 ml d'éthanol anhydre. On agite le mélange réactionnel à la température ambiante pendant 2 heures, puis on y ajoute 50 ml d'éthanol et, ensuite, une solution d'acide chlorhydrique en éthanol. On obtient ainsi par précipitation le chlorhydrate de 4-(3-trifluorométhylphényl)-1-(2-cyanoéthyl)-1,2,3,6-tétrahydropyridine identique au produit décrit à l'Exemple 2. Rendement: 69,9% de la théorie.

De la même façon, en faisant réagir la 4-(3-trifluorométhylphényl)-1,2,3,6-tétrahydropyridine avec la vinyl-méthylcétone, on obtient le chlorhydrate de 4-(3-trifluorométhylphényl)-1-(3-oxobutyl)-1,2,3,6-tétrahydropyridine identique au produit décrit à l'Exemple 6. Rendement: 60,2% de la théorie.

Dans les mêmes conditions opératoires, en faisant réagir la 4-(3-trifluorométhylphényl)-1,2,3,6-tétrahydropyridine avec le crotononitrile, on obtient le chlorhydrate de 4-(3-trifluorométhylphényl)-1-(2-cyano-1-méthyl)éthyl-1,2,3,6-tétrahydropyridine.

## Exemple 16

a) A une solution de 9,1 g de 4-(3-trifluorométhylphényl)-1,2,3,6-tétrahydropyridine et de 4,04 g de triéthylamine en 40 ml de chlorure de méthylène refroidie à 0°C, on ajoute, goutte à goutte, une solution de 4,2 g de chlorure de l'acide cyclopropanecarboxylique dans 20 ml de chlorure de méthylène tout en surveillant que la température ne dépasse pas 5°C. On abandonne le mélange réactionnel 30 minutes à 0—5°C, puis une heure à la température ambiante. On ajoute 200 ml d'éther diéthylique, on filtre, on lave la phase organique 3 fois à l'eau, on la sèche et on l'évapore à sec sous pression réduite. On obtient ainsi 11,5 g de 4-(3-trifluorométhylphényl)-1-cyclopropylcarbonyl-1,2,3,6-tétrahydropyridine; huile jaune/brun contenant de traces d'impurétés.

De la même façon, en faisant réagir la 4-(3-trifluorométhylphényl)-1,2,3,6-tétrahydropyridine avec, respectivement, le chlorure de cyclohexylcarbonyle, le chlorure de cyclohexylacétyle, le chlorure de 3-cyclohexylpropionyle, le chlorure de cyclopentylacétyle et le chlorure de cycloheptylacétyle en présence de triéthylamine, on obtient, respectivement:

- la 4-(3-trifluorométhylphényl)-1-cyclohexylcarbonyl-1,2,3,6-tétrahydropyridine, huile jaunâtre;
- la 4-(3-trifluorométhylphényl)-1-cyclohexylacétyl-1,2,3,6-tétrahydropyridine, huile jaunâtre;
- la 4-(3-trifluorométhylphényl)-1-(3-cyclohexylpropionyl-1,2,3,6-tétrahydropyridine, huile jaunâtre;
- la 4-(3-trifluorométhylphényl)-1-cyclopentylacétyl-1,2,3,6-tétrahydropyridine, huile brute; et
- la 4-(3-trifluorométhylphényl)-1-cycloheptylacétyl-1,2,3,6-tétrahydropyridine, huile impure.

b) A un mélange de 1,5 g de $LiAlH_4$ et 60 ml d'éther diéthylique à 25—30°C, on ajoute, en 30 minutes, 11 g de 4-(3-trifluorométhylphényl)-1-cyclopropylcarbonyl-1,2,3,6-tétrahydropyridine et 50 ml d'éther diéthylique anhydre. On abandonne 2 heures sous agitation à la température ambiante, puis on ajoute de l'eau, on sépare la phase éthérée par décantation, on la sèche sur du sulfate de sodium anhydre et on évapore le solvant sous pression réduite. On dissout l'huile résidue, constituée par la

4-(3-trifluorométhylphényl)-1-cyclopropylméthyl-1,2,3,6-tétrahydropyridine base, dans de l'isopropanol et on acidifie la solution avec du gaz chlorhydrique. On obtient ainsi le chlorhydrate de 4-(3-trifluorométhylphényl)-1-cyclopropylméthyl-1,2,3,6-tétrahydropyridine qui, après cristallisation de 50 ml d'acétone, fond à 178—180°C. Rendement: 46,5%.

De la même façon, on obtient:

- la 4-(3-trifluorométhylphényl)-1-cyclohexylméthyl-1,2,3,6-tétrahydropyridine, et son chlorhydrate qui fond à 217—220°C;
- la 4-(3-trifluorométhylphényl)-1-(2-cyclohexyléthyl)-1,2,3,6-tétrahydropyridine, et son chlorhydrate qui fond à 252—257°C;
- la 4-(3-trifluorométhylphényl)-1-(3-cyclohexylpropyl)-1,2,3,6-tétrahydropyridine, et son chlorhydrate qui fond à 219—222°C;
- la 4-(3-trifluorométhylphényl)-1-(2-cyclopentyléthyl)-1,2,3,6-tétrahydropyridine, et son chlorhydrate qui fond à 240—245°C; et
- la 4-(3-trifluorométhylphényl)-1-(2-cycloheptyléthyl)-1,2,3,6-tétrahydropyridine, et son chlorhydrate qui fond à 262—265°C.

## Exemple 17

A un mélange de 0,152 mole de $LiAlH(OCH_3)_3$ et 60 ml d'éther diéthylique anhydre à la température ambiante, on ajoute, en 30 minutes, 0,038 mole de 4-(3-trifluorométhylphényl)-1-(3-cyclohexylpropionyl)-1,2,3,6-tétrahydropyridine et 50 ml d'éther diéthylique anhydre. On abandonne 2 heures sous agitation à la température ambiante, puis on ajoute de l'eau, on décante la phase éthérée, on la sèche sur du sulfate de sodium anhydre et on évapore le solvant sous pression réduite. On dissout l'huile résidue, constituée par la 4-(3-trifluorométhylphényl)-1-(3-cyclohexylpropyl)-1,2,3,6-tétrahydropyridine base, en isopropanol et on acidifie la solution ainsi obtenue avec du gaz chlorhydrique. On obtient ainsi le chlorhydrate de 4-(3-trifluorométhylphényl)-1-(3-cyclohexylpropyl)-1,2,3,6-tétrahydropyridine qui, après cristallisation de l'acétone, fond à 219—222°C.

## Exemple 18

A un mélange de 0,114 mole de $AlH_3$ et 60 ml de tétrahydrofuranne anhydre on ajoute 0,038 mole de 4-(3-trifluorométhylphényl)-1-cycloheptylacétyl-1,2,3,6-tétrahydropyridine et 50 ml de tétrahydrofuranne. On chauffe au reflux 2 heures sous agitation, puis on refroidit, on ajoute de l'eau, on décante la phase éthérée, on la sèche sur du sulfate de sodium anhydre et on évapore le solvant sous pression réduite. On dissout l'huile résidue, constituée par la 4-(3-trifluorométhylphényl)-1-(2-cycloheptyléthyl)-1,2,3,6-tétrahydropyridine base, en isopropanol et on acidifie la solution ainsi obtenue avec du gaz chlorhydrique. On obtient ainsi le chlorhydrate de 4-(3-trifluorométhylphényl)-1-(2-cycloheptyléthyl)-1,2,3,6-tétrahydropyridine qui, après cristallisation dans de l'isopropanol, fond à 262—265°C.

## Exemple 19

On prépare des gélules à base de chlorhydrate de 4-(3-trifluorométhylphényl)-1-(2-cyanoéthyl)-1,2,3,6-tétrahydropyridine ayant la composition suivante:

| | |
|---|---|
| Principe actif | 15 mg |
| Lactose | 120 mg |
| Stéarate de magnésium | 5 mg |

en mélangeant intimement des charges des ingrédients ci-dessus et en versant le mélange dans des gélules de gélatine dure.

## Exemple 20

On prépare des comprimés à base de chlorhydrate de 4-(3-trifluorométhylphényl)-1-(2-cyanoéthyl)-1,2,3,6-tétrahydropyridine ayant la composition suivante:

| | |
|---|---|
| Ingrédient actif | 20 mg |
| Lactose | 100 mg |
| Cellulose microcristalline | 30 mg |

| | |
|---|---|
| Amidon de maïs séché | 40 mg |
| Stéarate de magnésium | 5 mg |

en broyant l'ingrédient actif jusqu'à une dimension particulaire de 0,4 mm, en le faisant passer à travers un tamis de 0,4 mm d'ouverture de maille, en mélangeant la matière broyée avec les autres constituants et en comprimant pour former les comprimés.

De la même façon, on prépare des comprimés contenant 40 mg d'ingrédient actif.

Exemple 21

En opérant comme décrit dans l'Exemple 20 ci-dessus, on prépare des comprimés ayant la composition suivante:

| | |
|---|---|
| Ingrédient actif | 50 mg |
| Lactose | 95 mg |
| Amidon de maïs | 100 mg |
| Talc | 4,5 mg |
| Stéarate de magnésium | 0,5 mg |

Exemple 22

10 000 gélules avec une teneur en substance active de 50 mg sont préparées à partir des constituants suivants: 500 g de chlorhydrate de 4-(3-trifluorométhylphényl)-1-(3-oxobutyl)-1,2,3,6-tétrahydropyridine, 495 g de cellulose microcristalline, 5 g de gel de silice amorphe. Les constituants ci-dessus sont bien mélangés et introduits dans des gélules de gélatine dure de dimension 4.

Exemple 23

On prépare une solution aqueuse stérile appropriée pour une utilisation parentérale en ampoules ayant la composition suivante:

| | |
|---|---|
| Chlorhydrate de 4-(3-trifluorométhylphényl)-1-(2-cyanoéthyl)-1,2,3,6-tétrahydropyridine | 30 mg |
| Chlorure de sodium | 5 mg |
| Eau distillée, q.s.p. | 2 ml |

Exemple 24

On prépare des suppositoires ayant la composition suivante:

| | |
|---|---|
| Chlorhydrate de 4-(3-trifluorométhylphényl)-1-(2-cyanoéthyl)-1,2,3,6-tétrahydropyridine | 50 mg |
| Lactose | 250 mg |
| Witepsol W 45, q.s.p. | 1,7 mg |

On mélange la substance active avec le lactose et on met le mélange uniformément en suspension dans la masse pour suppositoires fondue. On verse la suspension dans des moules refroidis pour former des suppositoires d'un poids de 1,7 g.

De la même façon, on prépare des suppositoires contenant

50 mg de chlorhydrate de 4-(3-trifluorométhylphényl)-1-cyanométhyl-1,2,3,6-tétrahydropyridine ou

50 mg de chlorhydrate de 4-(3-trifluorométhylphényl)-1-(3-cyanopropyl)-1,2,3,6-tétrahydropyridine.

Exemple 25

On prépare des comprimés ayant la composition suivante:

| | |
|---|---|
| Chlorhydrate de 4-(3-trifluorométhylphényl)-1-(2-cyano-1-méthyl)éthyl-1,2,3,6-tétrahydropyridine | 25 mg |
| Lactose | 95 mg |

| | |
|---|---|
| Amidon de maïs | 45 mg |
| Silice colloidale | 2 mg |
| Amidon soluble | 5 mg |
| Stéarate de magnésium | 3 mg |

On mélange la substance active avec une partie des adjuvants et on granule le mélange avec une solution de l'amidon soluble dans l'eau. Après séchage du granulat, on ajoute le reste des adjuvants et on forme des tablettes par compression.

De la même façon, on prépare des comprimés contenant

25 mg de chlorhydrate de 4-(3-trifluorométhylphényl)-1-cyanométhyl-1,2,3,6-tétrahydropyridine,
25 mg de chlorhydrate de 4-(3-trifluorométhylphényl)-1-(2-cyanoéthyl)-1,2,3,6-tétrahydropyridine,
25 mg de chlorhydrate de 4-(3-trifluorométhylphényl)-1-(3-cyanopropyl)-1,2,3,6-tétrahydropyridine, et, respectivement,
25 mg de chlorhydrate de 4-(3-trifluorométhylphényl)-1-(3-oxobutyl)-1,2,3,6-tétrahydropyridine.

## Exemple 26

On prépare des comprimés à base de chlorhydrate de 4-(3-trifluorométhylphényl)-1-(3-cyanopropyl)-1,2,3,6-tétrahydropyridine ayant la composition suivante:

| | |
|---|---|
| Ingrédient actif | 20 mg |
| Lactose | 100 mg |
| Cellulose microcristalline | 30 mg |
| Amidon de maïs séché | 40 mg |
| Stéarate de magnésium | 5 mg |

en utilisant le procédé décrit à l'Exemple 20.

De la même façon, on prépare des comprimés renfermant 40 mg d'ingrédient actif.

## Exemple 27

20 000 gélules contenant 50 mg d'ingrédient actif sont préparées à partir des constituants suivants: 1000 g de chlorhydrate de 4-(3-trifluorométhylphényl)-1-(3-cyclohexylpropyl)-1,2,3,6-tétrahydropyridine, 990 g de cellulose microcristalline et 10 g de gel de silice amorphe. Les constituants ci-dessus sont bien mélangés et introduits dans des gélules de gélatine dure de dimension 4.

De la même façon, on prépare des gélules contenant 50 mg de chlorhydrate de 4-(3-trifluorométhylphényl)-1-(2-cyclopentyléthyl)-1,2,3,6-tétrahydropyridine et, respectivement, 50 mg de chlorhydrate de 4-(3-trifluorométhylphényl)-1-(2-cyclohexyléthyl)-1,2,3,6-tétrahydropyridine.

**Revendications pour les Etats contractants: BE CH DE FR GB IT LI LU NL SE**

1. 4-(3-trifluorométhylphényl)-1,2,3,6-tétrahydropyridines de formule

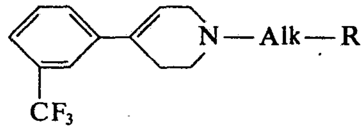

dans laquelle R représente un groupe cyano, un groupe acétyle ou groupe cycloalkyle ayant 3 à 7 atomes de carbone et Alk représente un groupe alkylène à chaîne droite ou ramifiée ayant de 1 à 4 atomes de carbone et leurs sels pharmaceutiquement acceptables.

2. Chlorhydrate de 4-(3-trifluorométhylphényl)-1-(2-cyanoéthyl)-1,2,3,6-tétrahydropyridine.
3. Chlorhydrate de 4-(3-trifluorométhylphényl)-1-(3-oxobutyl)-1,2,3,6-tétrahydropyridine.
4. Chlorhydrate de 4-(3-trifluorométhylphényl)-1-(2-cyclohexyléthyl)-1,2,3,6-tétrahydropyridine.
5. Chlorhydrate de 4-(3-trifluorométhylphényl)-1-(3-cyclohexylpropyl)-1,2,3,6-tétrahydropyridine.
6. Chlorhydrate de 4-(3-trifluorométhylphényl)-1-acétonyl-1,2,3,6-tétrahydropyridine.

7. Chlorhydrate de 4-(3-trifluorométhylphényl)-1-(2-cyano-1-methyl)éthyl-1,2,3,6-tétrahydropyridine.

8. Chlorhydrate de 4-(3-trifluorométhylphényl)-1-(3-cyanopropyl)-1,2,3,6-tétrahydropyridine.

9. Procédé pour la préparation de 4-(3-trifluorométhylphényl)-1,2,3,6-tétrahydropyridines de formule

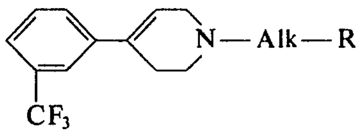

dans laquelle R représente un groupe cyano, acétyle ou cycloalkyle ayant 3 à 7 atomes de carbone et Alk représente un groupe alkylène à chaîne droite ou ramifiée ayant 1 à 4 atomes de carbone et de leurs sels pharmaceutiquement acceptables, caractérisé en ce qu'on fait réagir, dans un solvant organique à une température de 20 à 200°C, la 4-(3-trifluorométhylphényl)-1,2,3,6-tétrahydropyridine de formule

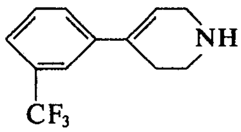

avec un composé de formule X — R, où R a la signification donnée ci-dessus et X représente un groupe chloro-, bromo-, ou iodoalkyle ayant 1 à 4 atomes de carbone ou bien, lorsque R est cyano ou acétyle, un groupe vinyle non substitué ou substitué par un ou deux groupes méthyle ou par un groupe éthyle.

10. Procédé selon la revendication 9 caractérisé en ce que le solvant organique est un alcool aliphatique contenant 1 à 6 atomes de carbone.

11. Procédé pour la préparation de 4-(3-trifluorométhylphényl)-1,2,3,6-tétrahydropyridines de formule

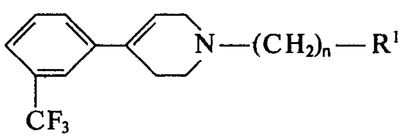

(IV)

dans laquelle $R^1$ représente un groupe cycloalkyle ayant 3 à 7 atomes de carbone et n représente 1, 2, 3 ou 4 et de leurs sels pharmaceutiquement acceptables caractérisé en ce qu'on réduit un composé de formule

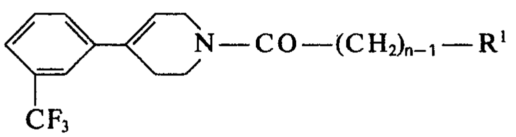

(V)

dans laquelle $R^1$ et n sont tels que définis ci-dessus, par l'hydrure d'aluminium ou par un hydrure complexe de lithium et d'aluminium dans un solvant organique inerte à une température de 0°C à la température d'ébullition du solvant employé et l'on transforme éventuellement le produit ainsi obtenu dans ses sels pharmaceutiquement acceptables.

12. Procédé selon la revendication 11 caractérisé en ce que comme agent de réduction on utilise l'hydrure de lithium et d'aluminium.

13. Procédé selon les revendications 11 et 12 caractérisé en ce que le composé V est obtenu en faisant réagir la 4-(3-trifluorométhylphényl)-1,2,3,6-tétrahydropyridine de formule

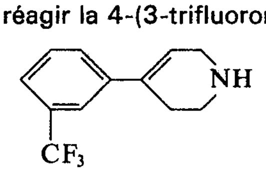

(III)

avec un dérivé fonctionnel d'un acide carboxylique de formule

$$R^1 - (CH_2)_{n-1} - COOH \qquad (VI)$$

où $R^1$ et n sont tels que définis dans la revendication 11, dans un solvant organique à une température comprise entre −10°C et la température d'ébullition du solvant employé.

14. Procédé selon la revendication 13 caractérisé en ce que comme dérivé fonctionnel de l'acide

carboxylique VI on utilise le chlorure et l'on opère en présence d'un accepteur de protons.

15. Procédé selon les revendications 13 et 14 caractérisé en ce que comme accepteur de protons on utilise la triéthylamine.

16. Procédé selon la revendication 13 caractérisé en ce que comme dérivé fonctionnel de l'acide carboxylique VI on utilise l'ester de p-nitrophényle.

17. Composition pharmaceutique à activité anorexigène caractérisée en ce qu'elle contient, en tant qu'ingrédient actif, un composé selon les revendications 1 à 8.

18. Composition pharmaceutique selon la revendication 16, sous forme d'unité de dosage, caractérisée en ce qu'elle contient de 1 à 500 mg d'ingrédient actif par unité de dosage en mélange avec un excipient pharmaceutique.

**Revendications pour l'Etat contractant: AT**

1. Procédé pour la préparation de 4-(3-trifluorométhylphényl)-1,2,3,6-tétrahydropyridines de formule

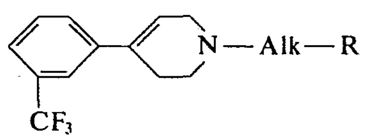

(I)

dans laquelle R représente un groupe cyano, acétyle ou cycloalkyle ayant 3 à 7 atomes de carbone et Alk représente un groupe alkylène à chaîne droite ou ramifiée ayant 1 à 4 atomes de carbone et de leurs sels pharmaceutiquement acceptables, caractérisé en ce qu'on fait réagir, dans un solvant organique à une température de 20 à 200°C, la 4-(3-trifluorométhylphényl)-1,2,3,6-tétrahydropyridine de formule

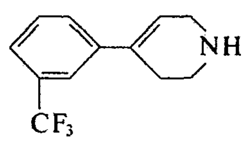

(III)

avec un composé de formule X — R, où R a la signification donnée ci-dessus et X représente un groupe chloro-, bromo-, ou iodoalkyle ayant 1 à 4 atomes de carbone ou bien, lorsque R est cyano ou acétyle, un groupe vinyle non substitué ou substitué par un ou deux groupes méthyle ou par un groupe éthyle.

2. Procédé selon la revendication 1 caractérisé en ce que le solvant organique est un alcool aliphatique contenant 1 à 6 atomes de carbone.

3. Procédé pour la préparation de 4-(3-trifluorométhylphényl)-1,2,3,6-tétrahydropyridines de formule

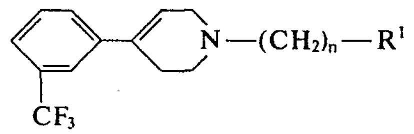

(IV)

dans laquelle $R^1$ représente un groupe cycloalkyle ayant 3 à 7 atomes de carbone et n représente 1, 2, 3 ou 4 et de leurs sels pharmaceutiquement acceptables caractérisé en ce qu'on réduit un composé de formule

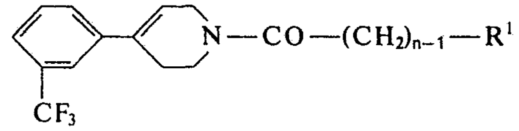

(V)

dans laquelle $R^1$ et n sont tels que définis ci-dessus, par l'hydrure d'aluminium ou par un hydrure complexe de lithium et d'aluminium dans un solvant organique inerte à une température de 0°C à la température d'ébullition du solvant employé et l'on transforme éventuellement le produit ainsi obtenu dans ses sels pharmaceutiquement acceptables.

4. Procédé selon la revendication 3 caractérisé en ce que comme agent de réduction on utilise l'hydrure de lithium et d'aluminium.

5. Procédé selon les revendications 3 et 4 caractérisé en ce que le composé V est obtenu en faisant réagir la 4-(3-trifluorométhylphényl)-1,2,3,6-tétrahydropyridine de formule

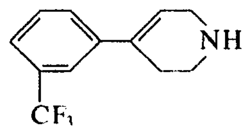 (III)

avec un dérivé fonctionnel d'un acide carboxylique de formule

$$R^1 - (CH_2)_{n-1} - COOH \qquad (VI)$$

où $R^1$ et n sont tels que définis dans la revendication 3 dans un solvant organique à une température comprise entre −10°C et la température d'ébullition du solvant employé.

6. Procédé selon la revendication 5 caractérisé en ce que comme dérivé fonctionnel de l'acide carboxylique VI on utilise le chlorure et l'on opère en présence d'un accepteur de protons.

7. Procédé selon les revendications 5 et 6 caractérisé en ce que comme accepteur de protons on utilise la triéthylamine.

8. Procédé selon la revendication 5 caractérisé en ce que comme dérivé fonctionnel de l'acide carboxylique VI on utilise l'ester de p-nitrophényle.

**Patentansprüche für die Vertragsstaaten: BE, CH, DE, FR, GB, IT, LI, LU, NL, SE**

1. 4-(3-Trifluormethylphenyl)-1,2,3,6-tetrahydropyridine der Formel

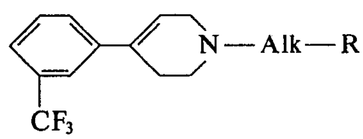

worin R eine Cyanogruppe, eine Acetylgruppe oder eine Cycloalkylgruppe mit 3 bis 7 Kohlenstoffatomen darstellt und Alk eine Alkylengruppe mit gerader oder verzweigter Kette mit 1 bis 4 Kohlenstoffatomen bedeutet, und ihre pharmazeutisch akzeptablen Salze.

2. 4-(3-Trifluormethylphenyl)-1-(2-cyanoäthyl)-1,2,3,6-tetrahydropyridin-hydrochlorid.

3. 4-(3-Trifluormethylphenyl)-1-(3-oxobuyl)-1,2,3,6-tetrahydropyridin-hydrochlorid.

4. 4-(3-Trifluormethylphenyl)-1-(2-cyclohexyläthyl)-1,2,3,6-tetrahydropyridin-hydrochlorid.

5. 4-(3-Trifluormethylphenyl)-1-(3-cyclohexylpropyl)-1,2,3,6-tetrahydropyridin-hydrochlorid.

6. 4-(3-Trifluormethylphenyl)-1-acetonyl-1,2,3,6-tetrahydropyridin-hydrochlorid.

7. 4-(3-Trifluormethylphenyl)-1-(2-cyano-1-methyl)-äthyl-1,2,3,6-tetrahydropyridin-hydrochlorid.

8. 4-(3-Trifluormethylphenyl)-1-(3-cyanopropyl)-1,2,3,6-tetrahydropyridin-hydrochlorid.

9. Verfahren zur Herstellung von 4-(3-Trifluormethylphenyl)-1,2,3,6-tetrahydropyridinen der Formel

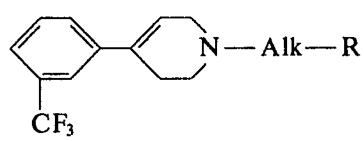

worin R eine Cyano-, Acetyl- oder Cycloalkylgruppe mit 3 bis 7 Kohlenstoffatomen darstellt und Alk eine Alkylengruppe mit gerader oder verzweigter Kette mit 1 bis 4 Kohlenstoffatomen bedeutet, und ihren pharmazeutisch akzeptablen Salzen, dadurch gekennzeichnet, daß man 4-(3-Trifluormethylphenyl)-1,2,3,6-tetrahydropyridin der Formel

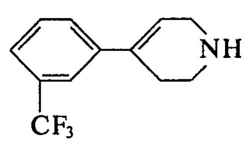

mit einer Verbindung der Formel X — R, worin R obige Bedeutung hat und X für eine Chlor-, Brom- oder Jodalkylgruppe mit 1 bis 4 Kohlenstoffatomen steht oder aber, wenn R Cyano oder Acetyl ist, für eine nicht substituierte oder durch eine oder zwei Methylgruppen oder durch eine Äthylgruppe substituierte Vinylgruppe steht, in einem organischen Lösungsmittel bei einer Temperatur von 20 bis 200°C zur Umsetzung bringt.

10. Verfahren nach Anspruch 9, dadurch gekennzeichnet, daß das organische Lösungsmittel ein aliphatischer Alkohol mit 1 bis 6 Kohlenstoffatomen ist.

11. Verfahren zur Herstellung von 4-(3-Trifluormethylphenyl)-1,2,3,6-tetrahydropyridinen der Formel

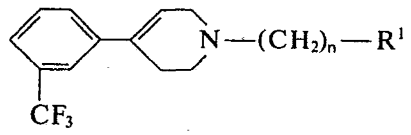

(IV)

worin $R^1$ eine Cycloalkylgruppe mit 3 bis 7 Kohlenstoffatomen darstellt und n für 1, 2, 3 oder 4 steht, und ihren pharmazeutisch akzeptablen Salzen, dadurch gekennzeichnet, daß man eine Verbindung der Formel

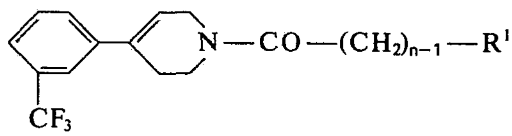

(V)

worin $R^1$ und n obige Bedeutung haben, mittels Aluminiumhydrid oder einem Lithium- und Aluminiumhydridkomplex in einem inerten organischen Lösungsmittel bei einer Temperatur von 0°C bis zur Kochtemperatur des verwendeten Lösungsmittels reduziert und gegebenenfalls das so erhaltene Produkt in seine pharmazeutisch akzeptablen Salze überführt.

12. Verfahren nach Anspruch 11, dadurch gekennzeichnet, daß man als Reduktionsmittel Lithium- und Aluminiumhydrid verwendet.

13. Verfahren nach den Ansprüchen 11 und 12, dadurch gekennzeichnet, daß die Verbindung V erhalten wird, indem man 4-(3-Trifluormethylphenyl)-1,2,3,6-tetrahydropyridin der Formel

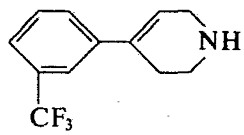

(III)

mit einem funktionellen Derivat einer Carbonsäure der Formel

$$R^1 - (CH_2)_{n-1} - COOH \qquad (VI)$$

worin $R^1$ und n die Bedeutung des Anspruchs 11 haben, in einem organischen Lösungsmittel bei einer Temperatur zwischen −10°C und der Kochtemperatur des verwendeten Lösungsmittels zur Umsetzung bringt.

14. Verfahren nach Anspruch 13, dadurch gekennzeichnet, daß man als funktionelles Derivat der Carbonsäure VI das Chlorid verwendet und in Gegenwart eines Protonenakzeptors arbeitet.

15. Verfahren nach Anspruch 14, dadurch gekennzeichnet, daß man als Protonenakzeptor Triäthylamin verwendet.

16. Verfahren nach Anspruch 13, dadurch gekennzeichnet, daß man als funktionelles Derivat der Carbonsäure VI p-Nitrophenylester verwendet.

17. Pharmazeutische Zusammensetzung mit anorexigener Wirkung, dadurch gekennzeichnet, daß sie als Wirkstoff eine Verbindung nach den Ansprüchen 1 bis 8 enthält.

18. Pharmazeutische Zusammensetzung nach Anspruch 17 in Dosiseinheitsform, dadurch gekennzeichnet, daß sie 1 bis 500 mg Wirkstoff pro Dosiseinheit in Mischung mit einem pharmazeutischen Exzipienten enthält.

**Patentansprüche für den Vertragsstaat: AT**

1. Verfahren zur Herstellung von 4-(3-Trifluormethylphenyl)-1,2,3,6-tetrahydropyridinen der Formel

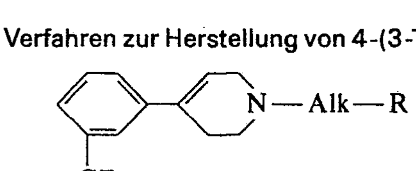

(I)

worin R eine Cyano-, Acetyl- oder Cycloalkylgruppe mit 3 bis 7 Kohlenstoffatomen darstellt und Alk eine Alkylengruppe mit gerader oder verzweigter Kette mit 1 bis 4 Kohlenstoffatomen bedeutet, und ihren pharmazeutisch akzeptablen Salzen, dadurch gekennzeichnet, daß man 4-(3-Trifluormethylphenyl)-1,2,3,6-tetrahydropyridin der Formel

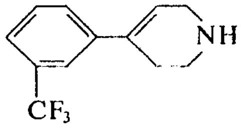

(III)

mit einer Verbindung der Formel X — R, worin R obige Bedeutung hat und X für eine Chlor-, Brom- oder Jodalkylgruppe mit 1 bis 4 Kohlenstoffatomen steht oder aber, wenn R Cyano oder Acetyl ist, für eine nicht substituierte oder durch eine oder zwei Methylgruppen oder durch eine Äthylgruppe substituierte Vinylgruppe steht, in einem organischen Lösungsmittel bei einer Temperatur von 20 bis 200°C zur Umsetzung bringt.

2. Verfahren nach Anspruch 1, dadurch gekennzeichnet, daß das organische Lösungsmittel ein aliphatischer Alkohol mit 1 bis 6 Kohlenstoffatomen ist.

3. Verfahren zur Herstellung von 4-(3-Trifluormethylphenyl)-1,2,3,6-tetrahydropyridinen der Formel

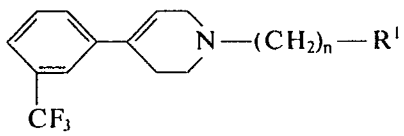

(IV)

worin $R^1$ eine Cycloalkylgruppe mit 3 bis 7 Kohlenstoffatomen darstellt und n für 1, 2, 3 oder 4 steht, und ihren pharmazeutisch akzeptablen Salzen, dadurch gekennzeichnet, daß man eine Verbindung der Formel

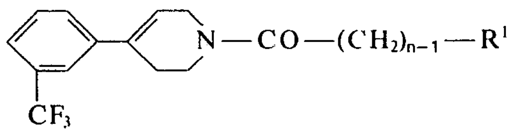

(V)

worin $R^1$ und n obige Bedeutung haben, mittels Aluminiumhydrid oder einem Lithium- und Aluminiumhydridkomplex in einem inerten organischen Lösungsmittel bei einer Temperatur von 0°C bis zur Kochtemperatur des verwendeten Lösungsmittels reduziert und gegebenenfalls das so erhaltene Produkt in seine pharmazeutisch akzeptablen Salze überführt.

4. Verfahren nach Anspruch 3, dadurch gekennzeichnet, daß man als Reduktionsmittel Lithium-und Aluminiumhydrid verwendet.

5. Verfahren nach den Ansprüchen 3 und 4, dadurch gekennzeichnet, daß die Verbindung V erhalten wird, indem man 4-(3-Trifluormethylphenyl)-1,2,3,6-tetrahydropyridin der Formel

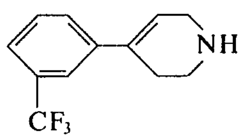

(III)

mit einem funktionellen Derivat einer Carbonsäure der Formel

$R^1 - (CH_2)_{n-1} - COOH$ (VI)

worin $R^1$ und n die Bedeutung des Anspruchs 3 haben, in einem organischen Lösungsmittel bei einer Temperatur zwischen −10°C und der Kochtemperatur des verwendeten Lösungsmittels zur Umsetzung bringt.

6. Verfahren nach Anspruch 5, dadurch gekennzeichnet, daß man als funktionelles Derivat der Carbonsäure VI das Chlorid verwendet und in Gegenwart eines Protonenakzeptors arbeitet.

7. Verfahren nach Anspruch 6, dadurch gekennzeichnet, daß man als Protonenakzeptor Triäthylamin verwendet.

8. Verfahren nach Anspruch 5, dadurch gekennzeichnet, daß man als funktionelles Derivat der Carbonsäure VI p-Nitrophenylester verwendet.

**Claims for the Contracting States: BE, CH, DE, FR, GB, IT, LI, LU, NL, SE**

1. 4-(3-trifluoromethylphenyl)-1,2,3,6-tetrahydropyridines of formula

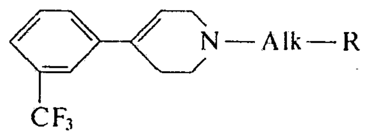

wherein R is a cyano group, an acetyl group or a cycloalkyl group of from 3 to 7 carbon atoms and Alk represents a straight or branched alkylene group of from 1 to 4 carbon atoms, and the pharmaceutically acceptable salts thereof.

2. The 4-(3-trifluoromethylphenyl)-1-(2-cyanoethyl)-1,2,3,6-tetrahydropyridine hydrochloride.

3. The 4-(3-trifluoromethylphenyl)-1-(3-oxobutyl)-1,2,3,6-tetrahydropyridine hydrochloride.

4. The 4-(3-trifluoromethylphenyl)-1-(2-cyclohexylethyl)-1,2,3,6-tetrahydropyridine hydrochloride.

5. The 4-(3-trifluoromethylphenyl)-1-(3-cyclohexylpropyl)-1,2,3,6-tetrahydropyridine hydrochloride.

6. The 4-(3-trifluoromethylphenyl)-1-acetonyl-1,2,3,6-tetrahydropyridine hydrochloride.

7. The 4-(3-trifluoromethylphenyl)-1-(2-cyano-1-methyl)ethyl-1,2,3,6-tetrahydropyridine hydrochloride.

8. The 4-(3-trifluoromethylphenyl)-1-(3-cyanopropyl)-1,2,3,6-tetrahydropyridine hydrochloride.

9. Process for the preparation of 4-(3-trifluoromethylphenyl)-1,2,3,6-tetrahydropyridines of formula

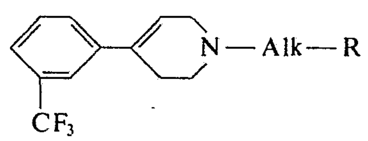

wherein R is a cyano group, an acetyl group or a cycloalkyl group of from 3 to 7 carbon atoms and Alk represents a straight or branched alkylene group of from 1 to 4 carbon atoms and of their pharmaceutically acceptable salts, characterized in that it consists in reacting the 4-(3-trifluoromethylphenyl)-1,2,3,6-tetrahydropyridine of formula

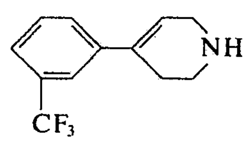

with a compound of formula X—R, in which R is as defined above and X is selected from the group consisting of chloro-, bromo-, and iodoalkyl groups having from 1 to 4 carbon atoms or, when R is a cyano or an acetyl group, an unsubstituted or with 1 or 2 methyl groups or with an ethyl group substituted vinyl group, in an organic solvent at a temperature of from 20°C to 200°C.

10. Process according to claim 9, characterized in that the organic solvent is an aliphatic alcohol having from 1 to 6 carbon atoms.

11. Process for the preparation of 4-(3-trifluoromethylphenyl)-1,2,3,6-tetrahydropyridines of formula

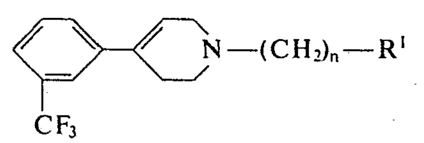

(IV)

wherein $R_1$ represents a cycloalkyl group of from 3 to 7 carbon atoms and n represents 1, 2, 3 or 4 and their pharmaceutically acceptable salts, characterized in that it consists in reducing a compound of formula

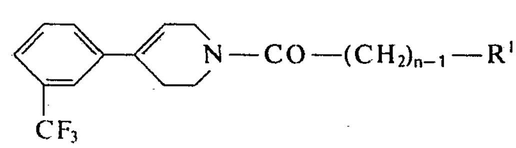

(V)

wherein $R_1$ and n are as defined above with aluminium hydride or an lithium and aluminium complex hydride in an inert organic solvent at a temperature of from 0°C to the boiling temperature of the used

solvent and possibly converting the obtained product into their pharmaceutically acceptable salts.

12. Process according to claim 11, characterized in that lithium aluminium hydride is used as reducing agent.

13. Process according to claims 11 and 12, characterized in that the compound V is obtained by reacting the 4-(3-trifluoromethylphenyl)-1,2,3,6-tetrahydropyridine of formula

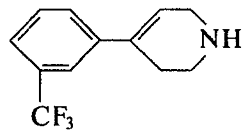

(III)

with a functionnal derivative of a carboxylic acid of formula

$$R^1 - (CH_2)_{n-1} - COOH \qquad (VI)$$

wherein $R^1$ and n are as defined in claim 11, in an organic solvent at a temperature of from −10°C to the boiling temperature of the used solvent.

14. Process according to claim 13, characterized in that the acid chloride is used as functionnal derivative of carboxylic acid IV and the reaction is carried out in the presence of a proton acceptor.

15. Process according to claims 13 and 14, characterized in that triethylamine is used as a proton acceptor.

16. Process according to claim 13, characterized in that the p-nitrophenyl ester is used as functionnal derivative of the carboxylic acid VI.

17. Pharmaceutical composition with anorexic activity characterized in that it contains, as active ingredient, a compound to claims 1 to 8.

18. Pharmaceutical composition according to claim 16 in dosage unit form characterized in that it contains from 1 to 500 mg of active ingredient in each dosage unit form in admixture with a pharmaceutical carrier.

**Claims for the Contracting States: AT**

1. Process for the preparation of 4-(3-trifluoromethylphenyl)-1,2,3,6-tetrahydropyridines of formula

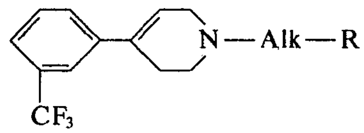

(I)

in which R is a cyano group, an acetyl group or a cycloalkyl group of from 3 to 7 carbon atoms and Alk represents a straight or branched alkylene group of from 1 to 4 carbon atoms and of their pharmaceutically acceptable salts, characterized in that it consists in reacting the 4-(3-trifluoromethylphenyl)-1,2,3,6-tetrahydropyridine of formula

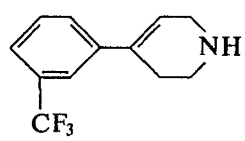

with a compound of formula X − R, in which R is as defined above and X is selected from the group consisting of chloro-, bromo-, and iodoalkyl groups having from 1 to 4 carbon atoms or, when R is a cyano or an acetyl group, an unsubstituted of with 1 or 2 methyl groups or with an ethyl group substituted vinyl group, in an organic solvent at a temperature of from 20°C to 200°C.

2. Process according to claim 1, characterized in that the organic solvent is an aliphatic alcohol having from 1 to 6 carbon atoms.

3. Process for the preparation of 4-(3-trifluoromethylphenyl)-1,2,3,6-tetrahydropyridines of formula

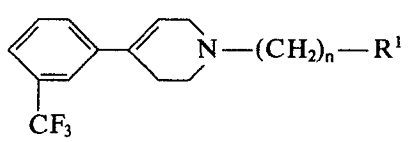

(IV)

wherein $R_1$ represents a cycloalkyl group of from 3 to 7 carbon atoms and n represents 1, 2, 3 or 4 and their pharmaceutically acceptable salts, characterized in that it consists in reducing a compound of formula

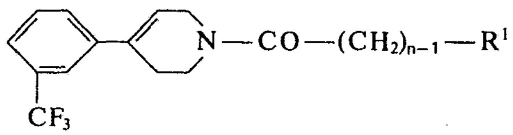

(V)

wherein $R_1$ and n are as defined above with aluminium hydride or an lithium and aluminium complex hydride in an inert organic solvent at a temperature of from 0°C to the boiling temperature of the used solvent and possibly converting the obtained product into their pharmaceutically acceptable salts.

4. Process according to claim 3, characterized in that lithium aluminium hydride is used as reducing agent.

5. Process according to claims 3 and 4, characterized in that the compound V is obtained by reacting the 4-(3-trifluoromethylphenyl)-1,2,3,6-tetrahydropyridine of formula

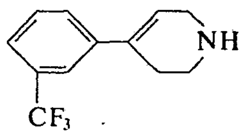

(III)

with a functionnal derivative of a carboxylic acid of formula

$$R^1 - (CH_2)_{n-1} - COOH \quad (VI)$$

wherein $R^1$ and n are as defined in claim 3, in an organic solvent at a temperature of from −10°C to the boiling temperature of the used solvent.

6. Process according to claim 5, characterized in that the acid chloride is used as functionnal derivative of carboxylic acid VI and the reaction is carried out in the presence of a proton acceptor.

7. Process according to claims 5 and 6, characterized in that triethylamine is used as a proton acceptor.

8. Process according to claim 5, characterized in that the p-nitrophenyl ester is used as functionnal derivative of the carboxylic acid VI.